# FASCICULE DE BREVET EUROPEEN

(11) **EP 2 177 450 B1**
(45) Date de publication et mention de la délivrance du brevet: **01.05.2013**
(21) Numéro de dépôt: 09173063.0
(22) Date de dépôt: 14.10.2009
(51) Int. Cl.: B65D 47/18, B65D 47/20, B65D 51/18, A61J 1/14, B05B 11/04, B05B 11/00

(54) **Dispositif de distribution de liquide muni d'un organe d'étanchéité déplaçable sous l'effet de la pression d'un utilisateur**
Vorrichtung zur Verteilung von Flüssigkeit, die mit einem unter dem Druckeffekt eines Benutzers verschiebbaren Dichtungsorgan ausgestattet ist
Liquid distribution device equipped with a sealing element that can move under the effect of pressure from a user

(30) Priorité: 15.10.2008 FR 0805715
(43) Date de publication de la demande: 21.04.2010
(73) Titulaire: Rexam Pharma La Verpillière, 38292 La Verpillière Cedex (FR)
(72) Inventeur: Painchaud, Gaetan, 69340, FRANCHEVILLE (FR); Grevin, Guillaume, 38080, L'ISLE D'ABEAU (FR); Julia, Xavier, 38090, VILLEFONTAINE (FR); Lanzi, Sylvain, 38850, CHIRENS (FR)
(74) Mandataire: Thiollier, Clémence-Olivia Laure Marie

(56) Documents cités:
- EP-A- 1 661 818
- WO-A-01/62618
- WO-A-02/068287
- FR-A- 1 434 743
- FR-A- 2 838 108
- GB-A- 2 122 586
- US-A- 2 628 004
- US-A- 5 226 568

## Description

La présente invention concerne le domaine technique de la distribution de liquide. En particulier, mais non exclusivement, elle concerne le domaine de la distribution de liquide sous forme de gouttes, tel que du liquide ophtalmique.

On connaît déjà dans l'état de la technique des dispositifs permettant de mettre en oeuvre cette distribution. Selon un exemple décrit dans le document FR 2 872 137, l'orifice de distribution de liquide peut être fermé de façon étanche grâce à un capuchon muni d'un picot pénétrant à l'intérieur de la buse de la tête de distribution et d'une jupe coiffant cette buse. Un tel capuchon permet d'éviter que du liquide s'échappe du dispositif lorsque ce dernier n'est pas utilisé.

Le document US 5 226 568 divulgue un dispositif selon le préambule de la revendication 1.

La présente invention propose notamment de fournir un dispositif assurant une meilleure étanchéité de l'embout de distribution lorsque le dispositif n'est pas utilisé, en particulier lors du transport ou du stockage du dispositif.

A cet effet, l'invention a pour objet un dispositif de distribution de liquide selon la revendication 1.

Différents modes de réalisation du dispositif de distribution de liquide selon l'invention sont définis par les revendications 2 à 9.

Ainsi, l'invention permet d'assurer une bonne étanchéité du dispositif dans le cas où ce dispositif comprend un tel organe d'étanchéité. Cet organe assurant l'étanchéité peut être aussi désigné "valve d'étanchéité". Il comprend généralement un matériau élastomère ou thermoplastique d'une souplesse suffisante et est destiné à faire passer du liquide uniquement dans un sens, par exemple lorsque l'utilisateur exerce une pression sur le réservoir. On notera que la position de libération de liquide et la position de blocage de liquide - ou position anti-retour - peuvent être prises par déplacement de l'organe et/ou par déformation élastique de tout ou partie de l'organe.

En immobilisant l'organe d'étanchéité dans sa position de blocage du liquide, on garantit que l'organe d'étanchéité ne prendra pas accidentellement sa position de libération, ce qui est particulièrement avantageux lors du stockage, du transport ou d'autres manipulations du dispositif avant utilisation, par exemple par un enfant. Ainsi, pour un dispositif muni d'un organe d'étanchéité mobile, il est intéressant d'immobiliser momentanément l'organe, grâce à des moyens d'immobilisation provisoire, ces moyens étant par exemple amovibles ou fusibles, les moyens fusibles pouvant être rompus au moment de la première utilisation du dispositif.

Outre le fait que l'on garantit que du liquide ne s'échappe pas du dispositif, on fournit un dispositif plus "propre". En effet, pendant le stockage, si l'organe n'est pas immobilisé, du liquide peut passer accidentellement entre l'organe d'étanchéité et un capuchon du dispositif, et stagner dans ce volume. Or, la présence de liquide hors de la zone d'étanchéité (délimitée notamment par le réservoir et l'organe d'étanchéité) est néfaste puisque le liquide est ainsi soumis à des contaminations.

Un dispositif peut en outre comporter l'une ou plusieurs des caractéristiques suivantes.
- Les moyens d'immobilisation exercent un appui sur l'organe d'étanchéité, de façon à l'empêcher de prendre sa position de libération de liquide. Cet appui peut être direct ou indirect.
- Le dispositif comporte une enveloppe extérieure coiffant notamment l'organe d'étanchéité, comprenant un orifice de libération du liquide, l'organe d'étanchéité comprenant une extrémité distale dépassant de l'orifice de libération de liquide. Ainsi, comme l'extrémité de l'organe d'étanchéité n'est pas en retrait par rapport à la surface de l'orifice de libération du liquide, il est plus facile d'isoler les gouttes de la surface de l'enveloppe extérieure.
- Le dispositif comporte en outre des moyens d'appui sur l'organe d'étanchéité, par exemple ménagés sur un noyau intérieur de l'embout, exerçant sur cet organe un appui opposé à l'appui exercé par les moyens d'immobilisation. Ainsi, lorsque les moyens d'immobilisation sont activés sur le dispositif, cet appui dans des directions opposées empêche l'organe d'étanchéité de bouger dans le dispositif de distribution. En d'autres termes, l'organe d'étanchéité est coincé par pincement entre les moyens d'immobilisation et les moyens d'appui.
- Le dispositif comporte un capuchon amovible et les moyens d'immobilisation sont activés lorsque le capuchon est monté sur le dispositif. Ainsi, le montage du capuchon sur le dispositif assure que l'organe d'étanchéité est immobilisé. Cette forme d'immobilisation est aisée à mettre en oeuvre, le capuchon étant généralement monté sur le dispositif dans les cas où l'on risque de libérer accidentellement du liquide, en particulier lors du transport ou du stockage, avant mise en vente du dispositif ou chez un particulier.
- Les moyens d'immobilisation sont portés par le capuchon.
- Les moyens d'immobilisation portés par le capuchon comportent une surface d'appui sur l'organe d'étanchéité. Selon un exemple cette surface d'appui est portée par une saillie, faisant saillie par rapport à une surface intérieure du capuchon, destinée à appuyer sur l'organe d'étanchéité. Cette saillie peut prendre différentes formes : elle peut comprendre une rainure annulaire formée sur la surface intérieure du capuchon, cette rainure annulaire pouvant éventuellement être coupée en secteurs séparés par des espaces, ou encore la saillie peut comprendre un pion pénétrant à l'intérieur de l'embout, dans un canal de passage de liquide, et venant appuyer sur l'organe d'étanchéité. Selon un autre exemple, la surface d'appui n'est pas saillante et est composée par la surface intérieure du capuchon. Selon encore un autre exemple, la surface d'appui correspond au fond d'un évidement réalisé à l'intérieur du capuchon. La surface d'appui est disposée au droit ou en quinconce par rapport aux moyens d'appui décrits plus haut, ménagés sur un noyau intérieur, et exerçant une force opposée à ces moyens d'appui. Ainsi l'étanchéité est assurée par compression et/ou déformation de l'organe d'étanchéité.
- Le dispositif comporte des moyens d'évent destinés à faire passer l'air à l'intérieur d'un réservoir. L'éventation du réservoir, ou passage d'air dans le réservoir, permet de combler une dépression générée par l'appui d'un utilisateur pour faire sortir du liquide. Le réservoir peut ainsi reprendre sa forme initiale après la distribution de liquide.
- Le capuchon comporte, outre les moyens d'immobilisation de l'organe d'étanchéité, des moyens de fermeture hermétique du dispositif, empêchant que de l'air passe dans les moyens d'évent lorsque le capuchon est monté sur le dispositif. Ce mode de réalisation est particulièrement avantageux. On peut ainsi éviter que du liquide s'évapore lors du stockage du dispositif, ou encore qu'il soit contaminé par des composants de l'air.
- Les moyens de fermeture hermétique du dispositif comprennent une nervure annulaire faisant saillie à l'intérieur d'une jupe du capuchon et venant en serrage contre une paroi pour assurer la fermeture hermétique.
- Le dispositif comporte une enveloppe extérieure coiffant notamment et au moins partiellement l'organe d'étanchéité, cette enveloppe comprenant un orifice de libération du liquide, et la surface d'appui des moyens d'immobilisation étant disposée au droit ou à l'intérieur de cet orifice lorsque le capuchon est monté sur le dispositif.
- L'enveloppe extérieure comprend une protubérance intérieure ouverte destinée à entourer l'extrémité distale de l'organe d'étanchéité. Cette protubérance intérieure assure ainsi un guidage de l'extrémité distale de l'organe d'étanchéité. En particulier, elle empêche l'organe d'étanchéité de se désaxer par rapport à son support au cours de son passage en position de libération ou en position de blocage.
- La protubérance intérieure ouverte est formée par une rainure annulaire centrale débouchant sur l'orifice de libération de liquide. Cette rainure annulaire peut ainsi entourer l'extrémité distale de l'organe d'étanchéité pour assurer son guidage.
- L'enveloppe extérieure comporte un siège d'appui d'un élément de rappel maintenant l'organe d'étanchéité en position de blocage, ce siège étant disposé autour de la protubérance intérieure ouverte.
- L'organe d'étanchéité est configuré pour prendre sa position de libération du liquide sous l'effet d'une pression créée par une action d'un utilisateur et les moyens d'immobilisation empêchent l'organe d'étanchéité de prendre sa position de libération de liquide sous l'effet d'une telle pression créée par une action de l'utilisateur.

L'invention sera mieux comprise à la lecture de la description qui va suivre, donnée uniquement à titre d'exemple et faite en se référant aux dessins dans lesquels :
- la figure 1 est une vue schématique illustrant fonctionnellement un dispositif ;
- la figure 2 est une vue en coupe longitudinale illustrant un exemple de dispositif tel que schématisé sur la figure 1 ;
- la figure 3 est une vue analogue à la figure 2, illustrant une variante de l'exemple de la figure 2 ; et
- la figure 4 est une vue analogue à la figure 2, illustrant un dispositif selon l'invention, une autre variante de l'exemple de la figure 2.

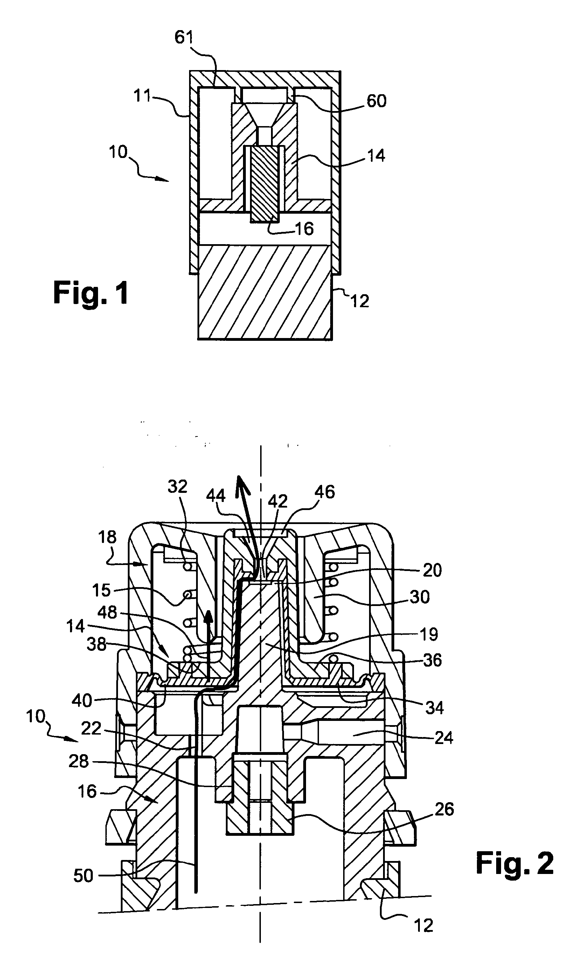

Un dispositif de distribution de liquide comprend un embout 10, représenté partiellement et schématiquement sur la figure 1, destiné à être rapporté sur un réservoir qui peut être en matière plastique, ou encore en verre ou en métal, contenant le liquide à distribuer. Le dispositif permet de distribuer des doses prédéterminées de liquide, plus précisément des gouttes de liquide ou un spray. Le liquide est destiné à une application oculaire, nasale ou buccale. Par exemple le dispositif distribue des gouttes de collyre pour les yeux. L'embout 10 est rapporté sur un réservoir 12, plus précisément sur le col 12 du réservoir. Dans cet exemple, le réservoir est en matière plastique et est destiné à être pressé par l'utilisateur pour faire sortir le liquide. On peut envisager d'autres types de réservoir, l'utilisateur pouvant libérer du liquide par une autre action qu'en le pressant, par exemple en appuyant sur un élément d'activation d'une pompe.

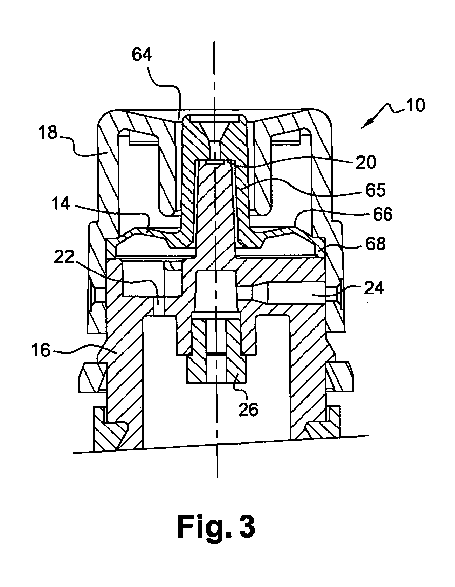

L'embout 10 comprend un organe d'étanchéité 14, disposé entre une première partie 16 et une deuxième partie 18 de l'embout 10. Cet organe d'étanchéité 14, ou valve d'étanchéité, peut prendre une position de blocage du liquide, ou position anti-retour, illustrée sur les figures 1, 2 et 3, empêchant le retour du liquide une fois sorti de la zone étanche, et une position de libération du liquide (non représentée). De préférence, l'organe 14 est disposé au voisinage de l'extrémité distale de l'embout 10, au voisinage d'un orifice 64 de libération de liquide (voir figure 3). Plus précisément, dans l'exemple décrit, l'extrémité distale de l'organe 14, extrémité par laquelle le liquide est délivré, dépasse légèrement de l'orifice 64. On pourrait envisager que cette extrémité arrive à affleurement de la surface de l'orifice 64, ou soit en retrait à l'intérieur de l'embout 10. Ou encore, on pourrait envisager que l'extrémité de l'organe 14 dépasse davantage de la surface de l'enveloppe 18, ce qui permet d'isoler plus facilement les gouttes par rapport à la surface de l'enveloppe 18.

Par ailleurs, l'embout 10 est recouvert d'un capuchon de fermeture 11, monté amovible sur le dispositif de façon à protéger le dispositif lorsqu'il n'est pas utilisé. Le capuchon est par exemple monté par vissage sur le col du réservoir 12.

Le capuchon 11 porte des moyens 60 d'immobilisation de l'organe d'étanchéité 14 dans sa position de blocage, configurés pour empêcher l'organe d'étanchéité de prendre sa position de libération de liquide, notamment sous l'effet d'une pression exercée sur le réservoir 12. Les moyens d'immobilisation 60 sont activés lorsque le capuchon 11 est monté sur le dispositif. Ils comportent, dans cet exemple, une surface d'appui, portée par une saillie 60, destinée à appuyer sur l'organe d'étanchéité 14, plus précisément une rainure annulaire 60 formée sur la surface intérieure 61 du capuchon 11, au droit de l'orifice 64 de libération de liquide de l'embout 10.

La configuration de l'embout 10 selon un mode de réalisation particulier va être décrit plus précisément, en références aux figures 2 et 3. L'exemple de la figure 3 correspond à une légère variante de celui de la figure 2. En effet, sur la figure 2, l'organe d'étanchéité 14 est en deux parties 34, 36 et est maintenu en position de blocage à l'aide d'un élément de rappel 15, alors que sur la figure 3, l'organe 14 est intégralement réalisé en matériau élastomère et est maintenu en position de blocage par déformation, comme cela est décrit dans la suite.

Comme cela est représenté sur la figure 2, la première partie 16 de l'embout 10 est un noyau intérieur 16, comprenant une protubérance 19 de forme sensiblement cylindrique et faisant saillie de l'extrémité distale du noyau 16. Sur son extrémité distale, cette protubérance 19 porte des moyens 20 d'appui sur l'organe d'étanchéité 14, ces moyens 20 étant dans cet exemple composés d'une saillie formant un bourrelet annulaire destinée à appuyer sur l'organe d'étanchéité 14. De façon alternative, les moyens d'appui 20 pourraient être composés de l'extrémité seule de la protubérance 19, sans inclure une saillie sur cette extrémité. Le noyau intérieur 16 comporte par ailleurs un canal 22 de passage du liquide depuis le réservoir vers l'extérieur du dispositif, ainsi qu'une partie de connexion du noyau 16 au réservoir 12. L'embout 10 comporte par ailleurs des moyens d'évent, destinés à faire passer de l'air à l'intérieur du réservoir pour combler une dépression générée par l'appui de l'utilisateur pour faire sortir du liquide. Dans cet exemple, les moyens d'évent sont portés par le noyau intérieur 16 et comprennent un canal 24 de passage de l'air, traversé par un filtre hydrophobe 26, destiné à filtrer l'air entrant sans pour autant permettre à du liquide de s'échapper par le canal 24. Plus précisément dans cet exemple, le filtre 26 est disposé dans un logement 28 de réception du filtre, ce logement 28 prenant la forme d'une rainure annulaire disposée au centre de la surface intérieure du noyau 16, dans laquelle le filtre 26 est encastré.

La deuxième partie 18 de l'embout 10 correspond, dans cet exemple, à une enveloppe supérieure extérieure de l'embout 10. Cette enveloppe extérieure 18 est destinée à coiffer le noyau intérieur 16, l'organe d'étanchéité 14 et le ressort 15. Plus précisément, elle comprend une protubérance intérieure ouverte 30, formée par une rainure annulaire centrale débouchant sur l'orifice 64, destinée à entourer l'extrémité distale de la protubérance 18 et de l'organe d'étanchéité 14 de façon à permettre le passage de liquide hors du dispositif. L'enveloppe 18 comporte en outre un siège 32 d'appui de l'élément de rappel 15, ce siège 32 étant disposé autour de la protubérance 30.

L'organe d'étanchéité comporte dans cet exemple une partie élastomère 34 et une partie rigide 36, les parties 34 et 36 étant solidaires l'une de l'autre en déplacement, c'est-à-dire que lorsque la partie 34 se déplace, la partie 36 se déplace également avec elle, et vice-versa. Dans cet exemple, les parties 34 et 36 sont assemblées par surmoulage, mais l'on pourrait envisager d'autres types d'assemblage. La partie élastomère 34 est réalisée dans un matériau élastomère, tel que du silicone ou un matériau thermoplastique élastomère. La partie rigide 36 est réalisée dans un matériau plastique tel que du polypropylène. La partie rigide 36 comporte une surface 38 d'appui de l'élément de rappel 15. Comme on peut le constater sur les figures, la partie rigide 36 recouvre la partie élastomère 34 sur sensiblement toute sa surface, une zone 40 de la partie élastomère étant néanmoins laissée libre à l'extrémité de la partie élastomère, de façon à permettre l'allongement de cette partie élastomère 34. Plus précisément, la partie élastomère 34, ainsi que la partie rigide 36, ont chacune la forme d'un chapeau muni d'une forme centrale cylindrique, de forme sensiblement complémentaire à celle de la protubérance 19 du noyau 16, cette forme cylindrique étant prolongée sur son extrémité proximale par un rebord. Ainsi, la partie rigide 36 recouvre la partie élastomère 34 sur une grande partie de sa surface, sauf au niveau de sa périphérie 40. Comme on peut le voir sur les figures, les parties 34, 36 définissent un canal 42, ménagé sur le fond de leur forme centrale cylindrique, permettant au liquide de sortir. Par ailleurs, la partie rigide 36 comporte des moyens 44 de formation de gouttes de liquide. Plus précisément, ces moyens 44 ont la forme d'un cône partant du canal 42 et s'élargissant vers l'extrémité distale du dispositif, de façon à former une goutte et à éviter que le liquide soit distribué par jet, le cône 44 débouchant sur une portion cylindrique 46, permettant de calibrer la goutte.

L'élément de rappel 15 est, dans cet exemple, un ressort métallique en spirale. Cet élément 15 exerce une force de rappel sur l'organe d'étanchéité 14, en s'appuyant sur la surface 38 de la partie rigide 36, de façon à rappeler l'organe d'étanchéité 14 dans sa position de blocage du liquide.

Comme on peut le voir sur les figures, l'organe d'étanchéité 14 est fixé entre les deux parties 16, 18, de façon étanche (étanchéité statique), afin d'éviter que du liquide, passant par le canal 22, ne s'échappe à l'intérieur de l'enveloppe 18.

Le fonctionnement du dispositif de distribution de la figure 2 va à présent être décrit.

Lorsque le capuchon 11 est monté sur le dispositif, les moyens d'immobilisation 60 sont activés et l'organe 14 est donc immobilisé. Plus précisément les moyens d'appui 20 exercent sur l'organe 14 un appui opposé à l'appui exercé par les moyens d'immobilisation 60. Il en résulte que l'organe 14 est comprimé contre le noyau 16, assurant l'étanchéité.

Lorsque l'utilisateur souhaite utiliser le dispositif, il ôte tout d'abord le capuchon 11, ce qui désactive les moyens d'immobilisation 60, et rend donc l'organe 14 mobile entre ses deux positions. Pour distribuer des gouttes de liquide, l'utilisateur presse sur le réservoir du dispositif, ce qui a pour effet de faire passer du liquide dans le canal 22, et donc d'exercer une pression sur la partie élastomère 34. Sous cette pression, l'organe d'étanchéité passe de sa position de blocage de liquide à sa position de libération de liquide, en effectuant une translation vers le haut, comme illustré par la flèche 48. Plus précisément, la zone 40 de la partie élastomère 34 se déforme, en s'allongeant, pour autoriser ce déplacement vers le haut de la partie élastomère. A l'issue de ce déplacement, l'étanchéité assurée par la coopération des moyens d'appui 20 avec l'organe d'étanchéité 14 est rompue, et le liquide peut passer à travers le canal 42 jusque dans les parties 44, 46, de façon à former une goutte de liquide. Le trajet du liquide est illustré par la flèche 50. Une fois la goutte libérée, l'utilisateur peut arrêter d'exercer la pression sur le réservoir, lequel se remplit d'air grâce au passage au canal 24. Par ailleurs, la pression du liquide sortant s'arrêtant, l'organe d'étanchéité 14 reprend sa position de blocage du liquide, sous l'effet de la force de rappel de l'élément 15. Ainsi, les moyens d'appui 20 et la partie élastomère de l'organe 14 coopèrent à nouveau de façon à bloquer la sortie de liquide.

En remontant le capuchon sur l'embout, on peut à nouveau immobiliser l'organe 14 dans cette position, et éviter la contamination de liquide passé accidentellement hors de la zone étanche.

Parmi les avantages du dispositif de distribution de cet exemple, on notera en particulier que la partie rigide 36 constitue une sorte de coquille pour la partie élastomère 34, ce qui permet d'exercer plus facilement une contrainte sur l'organe d'étanchéité 14, sans risquer de le déformer.

On notera que l'exemple décrit peut présenter des variantes. En particulier, l'élément de rappel 15 est un ressort en spirale, mais on pourrait prévoir d'autres types d'éléments de rappel, en matière métallique ou pas, tels qu'une lame élastique ou un élément élastomère. Notamment, cet élément de rappel 15 pourrait être directement intégré dans l'organe d'étanchéité 14, en étant intégré soit dans la partie élastomère 34 soit dans la partie rigide 36, ou encore être directement intégré dans la partie 18.

Sur la variante de la figure 3, l'organe d'étanchéité est intégralement réalisé en matériau élastomère et l'embout ne présente pas de ressort. En effet dans cet exemple, l'organe 14 assure le blocage de liquide en étant monté déformé sur le dispositif. En effet l'organe 14 est en forme de chapeau, muni d'une forme cylindrique 65 et d'un rebord 66. La périphérie 68 de ce rebord est fixée de façon permanente entre les parties 16 et 18, de façon à assurer une étanchéité statique. Cette fixation est faite en déformant l'organe 14 : celui-ci est chaussé sur la protubérance 19 et déformé de façon que l'organe 14 est contraint de se plaquer contre la protubérance 19, plus précisément contre les moyens 20. En effet, la forme de l'organe 14 illustré sur la figure 3 est différente de la forme de l'organe 14 avant assemblage. Ce plaquage de l'organe 14 assure l'étanchéité dans la position de blocage.

Le fonctionnement du dispositif de la figure 3 est analogue à celui de la figure 2.

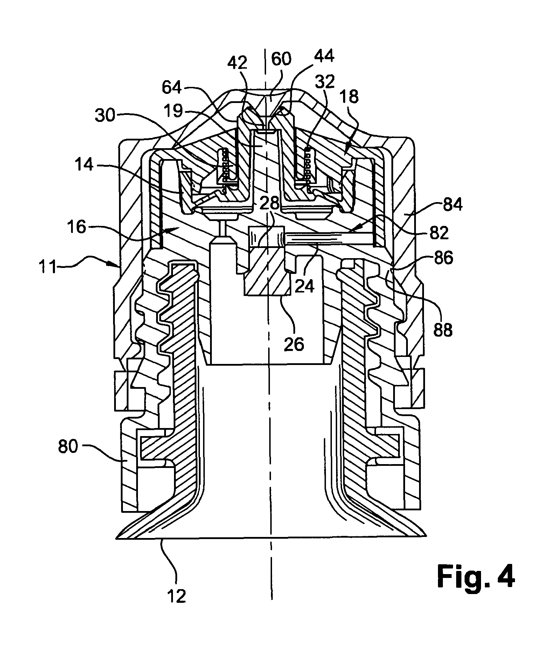

Sur la figure 4, la plupart des éléments sont analogues à ceux des figures 1 à 3. L'embout 10 est monté sur le col du réservoir 12 par vissage d'une partie de connexion de la partie 16 sur le col, cette partie comprenant ici une jupe extérieure 80 vissée sur le col. De même que pour les autres figures, la partie 16 comporte également des moyens d'évent 82, destinés à faire passer de l'air à l'intérieur du réservoir pour combler une dépression générée par l'appui de l'utilisateur pour faire sortir du liquide. Les moyens d'évent sont portés par le noyau intérieur 16 et comprennent les éléments 24, 26, 28. L'organe d'étanchéité est réalisé intégralement en matériau élastomère, mais l'on pourrait envisager qu'il comprenne également une partie rigide. Par ailleurs, sur la figure 4, l'organe d'étanchéité 14 dépasse encore davantage de l'orifice de libération 64, l'extrémité distale 19 du noyau 16 arrivant sensiblement à affleurement avec l'ouverture de l'orifice 64 réalisée à la surface supérieure de l'enveloppe 18. Ainsi, les gouttes se détachent plus facilement de la surface supérieure de l'enveloppe 18.

Le capuchon 11 est représenté sur la figure 4. Dans cet exemple, les moyens 60 d'immobilisation de l'organe 14 comprennent une saillie de forme sensiblement conique. Plus précisément elle est de forme sensiblement complémentaire des moyens 44 de formation de goutte, de façon à mieux venir immobiliser l'extrémité distale de l'organe 14, par pincement, lorsque le capuchon 11 est monté sur le dispositif. Le capuchon 11 comporte par ailleurs une jupe 84 de fixation du capuchon sur le dispositif. La jupe est fixée par vissage sur la partie 16. Néanmoins, elle pourrait être fixée par vissage sur le réservoir 12, ou encore être fixée sur l'un ou l'autre de ces éléments par encliquetage. La jupe 84 porte des moyens 86 de fermeture hermétique du dispositif, empêchant que de l'air passe dans les moyens d'évent 82 lorsque le capuchon 11 est monté sur le dispositif. Dans cet exemple, les moyens 86 comprennent une nervure annulaire faisant saillie à l'intérieur de la jupe 84 du capuchon et venant en serrage contre une paroi 88 de la partie 16 pour assurer la fermeture hermétique. En effet, la nervure 86 a un diamètre intérieur strictement inférieur au diamètre extérieur de la paroi 88, de sorte que, lorsque le capuchon 11 est vissé sur le dispositif, la nervure 86 est plaquée contre la paroi 88, empêchant ainsi tout air de passer entre les parties 86 et 88. Les parties 86, 88 définissent ainsi une zone d'étanchéité à l'air, disposée entre l'extrémité de la jupe 84 du capuchon et les moyens d'évent 82.

On notera que l'invention n'est pas limitée aux exemples précédemment décrits. En particulier, la forme de l'organe 14 peut varier, ainsi que la façon de l'immobiliser. Parmi les avantages du dispositif, on comprend que le capuchon 11, assurant l'immobilisation de l'organe 14, évite que du liquide s'infiltre dans les zones non étanches du dispositif, par exemple entre l'organe 14 et l'enveloppe 18, ou encore à la surface de l'embout 10.

## Revendications

1. Dispositif de distribution de liquide comportant un organe d'étanchéité (14) pouvant prendre une position de libération du liquide et une position de blocage du liquide, le dispositif comprenant en outre un capuchon amovible (11) et des moyens (60) d'immobilisation de l'organe d'étanchéité (14) dans sa position de blocage, configurés pour empêcher l'organe d'étanchéité de prendre sa position de libération de liquide, les moyens d'immobilisation (60) étant portés par le capuchon (11), exerçant un appui sur l'organe d'étanchéité et étant activés lorsque le capuchon est monté sur le dispositif, **caractérisé en ce qu'**il comporte une enveloppe extérieure (18) coiffant notamment l'organe d'étanchéité (14), l'enveloppe extérieure (18) comprenant une protubérance intérieure ouverte (30) ainsi qu'un orifice (64) de libération du liquide, l'organe d'étanchéité (14) comprenant une extrémité distale dépassant de l'orifice (64) de libération de liquide, et **en ce que** la protubérance (30) est destinée à entourer une partie de l'extrémité distale de l'organe d'étanchéité (14) de façon à assurer le guidage de l'organe d'étanchéité (14) au cours de son passage en position de libération ou de blocage du liquide.

2. Dispositif selon la revendication précédente, comportant des moyens d'évent destinés à faire passer l'air à l'intérieur d'un réservoir (12).

3. Dispositif selon la revendication précédente, dans lequel le capuchon (11) comporte, outre les moyens d'immobilisation de l'organe d'étanchéité, des moyens de fermeture hermétique du dispositif, empêchant que de l'air passe dans les moyens d'évent lorsque le capuchon (11) est monté sur le dispositif.

4. Dispositif selon la revendication précédente, dans lequel les moyens de fermeture hermétique du dispositif comprennent une nervure annulaire faisant saillie à l'intérieur d'une jupe (84) du capuchon (11) et venant en serrage contre une paroi pour assurer la fermeture hermétique.

5. Dispositif selon l'une quelconque des revendications précédentes, comportant des moyens (20) d'appui sur l'organe d'étanchéité (14), exerçant sur cet organe un appui opposé à l'appui exercé par les moyens d'immobilisation (60).

6. Dispositif selon l'une quelconque des revendications précédentes, dans lequel les moyens d'immobilisation comportent une surface (60) d'appui sur l'organe d'étanchéité (14), par exemple une saillie (60) destinée à appuyer sur l'organe d'étanchéité (14), éventuellement une rainure annulaire formée sur la surface intérieure (61) du capuchon.

7. Dispositif selon les revendications 5 et 6, dans lequel la surface d'appui (60) des moyens d'immobilisation est disposée au droit ou en quinconce par rapport aux moyens d'appui (20).

8. Dispositif selon l'une quelconque des revendications précédente, dans lequel la protubérance intérieure ouverte (30) est formée par une rainure annulaire centrale débouchant sur l'orifice de libération de liquide (64).

9. Dispositif selon l'une quelconque des revendications précédente, dans lequel l'enveloppe extérieure (18) comporte un siège (32) d'appui d'un élément de rappel (15) maintenant l'organe d'étanchéité en position de blocage, ce siège (32) étant disposé autour de la protubérance intérieure ouverte (30).

## Claims

1. Liquid dispensing device comprising a sealing component (14) which can take up a liquid release position and a liquid blocking position, the device also comprising a removable cap (11) and means (60) for immobilising the sealing component (14) in its blocking position, configured to prevent the sealing component from taking up its liquid release position, the immobilisation means (60) being supported by the cap (11), exerting a pressure on the sealing component and being activated when the cap is fitted on the device, **characterized in that** the device comprises an outer envelope (18) covering in particular the sealing component (14), the outer envelope (18) comprising an inner opened projection (30) as well as a liquid release orifice (64), the sealing component (14) comprising a distal end projecting from the liquid release orifice, and **in that** the projection (30) is intended to encircle a part of the distal end of the sealing component (14), ensuring a guiding of the sealing component (14) during the movement into the liquid release or the blocking position.

2. Device according to the preceding claim, comprising venting means allowing air to enter into a reservoir (12).

3. Device according to the preceding claim, wherein the cap (11) comprises, in addition to the immobilisation means for the sealing component, means (86) for an airtight sealing of the device, preventing air from passing through the venting means when the cap (11) is fitted on the device.

4. Device according to the preceding claim, wherein the airtight sealing means comprise an annular rib projecting inside from a wall (84) of the cap (11) and being clamped against a wall for ensuring the airtight sealing.

5. Device according to any of the preceding claims, comprising means (20) for pressing on the sealing component (14), exerting on this component a pressure opposite to the pressure exerted by the immobilisation means (60).

6. Device according to any of the preceding claims, wherein the immobilisation means comprise a surface (60) for pressing on the sealing component (14), for example a projection (60) designed to press on the sealing component (14), possibly an annular rib formed on the inner surface (61) of the cap.

7. Device according to claims 5 and 6, wherein the pressing surface (60) of the immobilisation means is arranged opposite or staggered with respect to the pressing means (20).

8. Device according to any of the preceding claims, wherein the inner opened projection (30) is formed by an annular rib ending by the liquid release orifice (64).

9. Device according to any of the preceding claims, wherein the outer envelope (18) comprises a bearing seat (32) for a return element (15) holding the sealing component in blocking position, the seat (32) being arranged around the inner opened projection (30).

## Patentansprüche

1. Vorrichtung zur Verteilung von Flüssigkeit mit einem Dichtungsorgan (14), das eine Position zum Freisetzen von Flüssigkeit und eine Position zum Sperren von Flüssigkeit einnehmen kann, wobei die Vorrichtung außerdem eine abnehmbare Kappe (11) und Mittel (60) zum Befestigen des Dichtungsorgans (14) in seiner Sperrposition umfasst, die derart eingerichtet sind, dass das Dichtungsorgan daran gehindert wird, seine Freisetzungsposition für die Flüssigkeit einzunehmen, wobei die Befestigungsmittel (60) von der Kappe (11) getragen werden, die sich auf dem Dichtungsorgan abstützen und aktiviert werden, wenn die Kappe auf der Vorrichtung angebracht wird, **dadurch gekennzeichnet, dass** sie eine Außenhülle (18) umfasst, die insbesondere das Dichtungsorgan (14) bedeckt, wobei die Außenhülle (18) einen offenen Innenvorsprung (30) sowie eine Öffnung (64) zum Freisetzen von Flüssigkeit umfasst, wobei das Dichtungsorgan (14) ein entferntes Ende aufweist, das aus der Öffnung (64) für die Freisetzung der Flüssigkeit herausragt, und dass der Vorsprung (30) dazu dient, einen Abschnitt des entfernten Endes des Dichtungsorgans (14) derart zu umgeben, dass die Führung des Dichtungsorgans (14) bei seinem Übergang in die Position der Freisetzung oder Sperrung der Flüssigkeit sichergestellt ist.

2. Vorrichtung nach dem vorangehenden Anspruch, die Belüftungsmittel umfasst, die ausgelegt sind, Luft in das Innere eines Reservoirs (12) zu lassen.

3. Vorrichtung nach dem vorangehenden Anspruch, in welcher die Kappe (11) neben den Befestigungsmitteln für das Dichtungsorgan Mittel zum hermetischen Verschließen der Vorrichtung umfasst, die verhindern, dass Luft in die Belüftungsmittel eindringt, wenn die Kappe (11) auf der Vorrichtung angebracht ist.

4. Vorrichtung nach dem vorangehenden Anspruch, bei welcher die Mittel zum hermetischen Verschließen der Vorrichtung eine ringförmige Rippe umfasst, die nach innen von einem Mantel (84) der Kappe (11) vorspringt und gegen eine Wand drückt, so dass der hermetische Verschluss sichergestellt ist.

5. Vorrichtung nach einem der vorangehenden Ansprüche, die Mittel (20) zum Abstützen auf dem Dichtungsorgan (14) umfasst, die auf diesem Organ eine Stütze bilden gegenüber der Stütze, die durch Befestigungsmitteln (60) gebildet wird.

6. Vorrichtung nach einem der vorangehenden Ansprüche, bei der die Befestigungsmittel eine Fläche (60) zum Abstützen auf dem Dichtungsorgan (14) umfassen, beispielsweise einen Vorsprung (60), der ausgelegt ist zum Abstützen auf dem Dichtungsorgan (14), gegebenenfalls eine ringförmige Rippe auf der Innenfläche (61) der Kappe.

7. Vorrichtung nach Anspruch 5 und 6, bei der die Stützfläche (60) der Befestigungsmittel rechtwinklig oder versetzt in Bezug auf die Stützmittel (20) angeordnet ist.

8. Vorrichtung nach einem der vorangehenden Ansprüche, bei der der offene Innenvorsprung (30) durch eine ringförmige Rippe gebildet wird, die in der Öffnung Freisetzungsöffnung für die Flüssigkeit (64) mündet.

9. Vorrichtung nach einem der vorangehenden Ansprüche, bei der die Außenhülle (18) einen Sitz (32) zum Abstützen eines Rückholelements (15) umfasst, so dass das Dichtungsorgan in der Sperrposition gehalten wird, wobei der Sitz (32) um den offenen Innenvorsprung (30) herum angeordnet ist.
